# EUROPÄISCHE PATENTSCHRIFT

(11) **EP 0 407 897 B1**
(45) Veröffentlichungstag und Bekanntmachung des Hinweises auf die Patenterteilung: **01.05.1996**
(21) Anmeldenummer: 90112900.7
(22) Anmeldetag: 06.07.1990
(51) Int. Cl.: C07D 207/416

(54) **Verbindungen aus Biopolymeren und Effektorsubstanzen, die über optisch aktive Aminosäurederivaten verknüpft sind, Verfahren zu ihrer Herstellung und ihre Verwendung**
Compounds of biopolymers effector substances bonded together by optically active aminoacidic derivatives, process for their preparation and their use
Composés préparés à partir de biopolymères et de composés effecteurs, les derniers reliés entre eux par des dérivés d'acides aminés optiquement actifs, procédé pour leur préparation et leur application

(30) Priorität: 10.07.1989 DE 3922608
(43) Veröffentlichungstag der Anmeldung: 16.01.1991
(73) Patentinhaber: BEHRINGWERKE Aktiengesellschaft, D-35001 Marburg (DE)
(72) Erfinder: Friesen, Heinz-Jürgen, Dr., D-3550 Marburg (DE); Hermentin, Peter, Dr., D-3550 Marburg (DE)

## Beschreibung

Die Erfindung betrifft Verbindungen aus Biopolymeren und Effektorsubstanzen, die mit Hilfe von Derivaten von optisch aktiven Aminosäuren, in denen die Aminogruppe in eine Maleimidogruppe und die Carboxylgruppe in eine Aktivestergruppe umgewandelt worden ist, verknüpft werden.

Im diagnostischen und therapeutischen Bereich werden durch chemisch kovalente Verknüpfung herstellbare Konjugate aus Biopolymeren wie Peptiden oder Proteinen und Markierungs- oder Effektorsubstanzen oder spezifischen Bindungspartnern benötigt. Bevorzugt werden bei diesen Kopplungen Methoden eingesetzt, bei denen die spezifischen Eigenschaften der miteinander zu verknüpfenden Komponenten durch die Kopplung gar nicht oder aber in definierter Form verändert werden. Eine Übersicht über bekannte Methoden zur Herstellung von Proteinkonjugaten aus dem Bereich der Enzymimmunoassays ist in einem Reviewartikel von Ishikawa et al. gegeben (J. Immunoassay 24 (1983) 209 - 327). Methoden zur Herstellung von Immuntoxinen sind beispielsweise in einem Reviewartikel von Ghose et al. aufgelistet (Methods in Enzymology, Vol. 93 (1983) pp. 280 - 333). Verfahren zur Herstellung von Konjugaten unter Verwendung heterobifunktioneller Reagenzien ermöglichen die gezielte, kontrollierte Verknüpfung der Kopplungspartner. Bei Proteinkonjugaten spielen Methoden, bei denen Verküpfungen zwischen SH-Gruppen und Aminogruppen hergestellt werden, eine besondere Rolle wegen der geringen Gefahr zu Nebenreaktionen bei sachgerechter Reaktionsführung. Vernetzungsreagenzien mit Maleimido-, Haloalkyl- oder Haloacyl- und Aktivester-Strukturen werden dabei bevorzugt verwendet.

Die bisher beschriebenen Vernetzungsreagenzien waren, da sie kein chirales C-Atom in der Brücke aufweisen, nicht geeignet, Konjugate mit definierter chiraler Struktur in der Brücke zu bilden. Darüber hinaus war es nicht möglich, bei Vernetzungsreagenzien mit kurzkettigem Brückenteil, die Polarität des Verknüpfungsreagenzes den jeweils erforderlichen Bedingungen anzupassen.

Die der vorliegenden Erfindung zugrundeliegende Aufgabe bestand nun darin, für Diagnose und Therapie wichtige Konjugate aus Biopolymeren und Effektorsubstanzen in einer sterisch definierten Form zur Verfügung zu stellen, wobei das Verknüpfungsreagenz in Bezug auf Länge, Beweglichkeit und Polarität den für die zu koppelnden Komponenten optimalen Kopplungsbedingungen angepaßt werden muß. Weiterhin sollen die bei den Kopplungen gebildeten Brücken genügende Stabilität aufweisen und gegebenenfalls vorher bestimmbar spaltbar sein.

Die vorliegende Erfindung betrifft Konjugate der Formel I 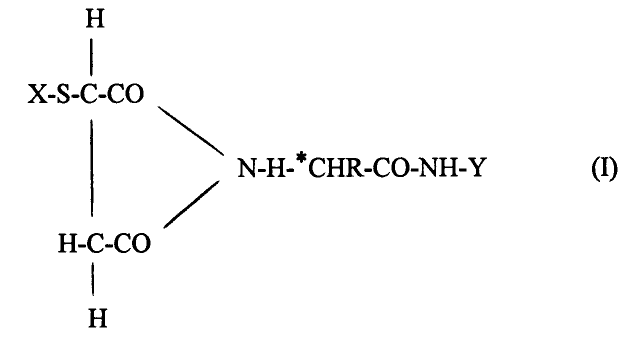
worin *C ein asymmetrisches Kohlenstoffatom ist, R für die Seitenkette einer natürlichen Aminosäure, des Methioninsulfons oder der Cysteinsäure steht,
X für den Rest der Thiolkopplungskomponente und
Y für den Rest der Aminokopplungskomponente steht, wobei zur Kopplung eine Verbindung der Formel (II) eingesetzt wird.

Bevorzugt sind dabei Verbindungen, worin die Thiol-Kopplungskomponente X-SH und die Aminokopplungskomponente Y-NH₂ jeweils Proteine sind.

Besonders bevorzugt sind Verbindungen, worin
X-SH ein Enzym, einen Antikörper oder ein Antikörperfragment und
Y-NH₂ ein Enzym, einen Antikörper oder ein Antikörperfragment bedeuten.

Ganz besonders bevorzugt sind Verbindungen, in denen das Enzym Peroxidase, β-Galactosidase oder Phosphatase ist und die Antikörper oder Antikörperfragmente vom Kaninchen, vom Schaf, von der Ziege oder der Maus stammen, insbesondere bevorzugt sind dabei Antikörper oder Antikörperfragmente gegen CEA, AFP, HCG, TSH und HBsAg.

Bevorzugt sind außerdem Verbindungen, worin R die Seitenkette der Aminosäure Alanin, des Methioninsulfons oder der Cysteinsäure und ganz besonders bevorzugt der Aminosäure Alanin bedeutet.

Ferner betrifft die Erfindung ein Verfahren zur Herstellung einer Verbindung nach Formel I, wobei in einem ersten Reaktionsschritt durch Umsetzung einer Aminokomponente 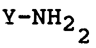
mit der funktionellen Aktivestergruppe einer Verbindung der Formel II 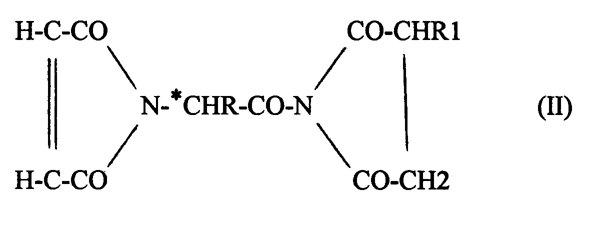
worin *C ein asymmetrisches Kohlenstoffatom ist,
R die zu Formel I angegebene Bedeutung hat und
R¹ Wasserstoff oder einen Rest der Formel III bedeutet, 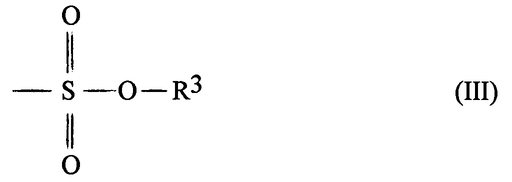
worin R³ Wasserstoff oder ein 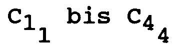
Alkyl ist, ein Amid und in einem zweiten Reaktionsschritt durch Umsetzung einer Thiolkomponente X-SH mit der Maleimido gruppe der Verbindung der Formel II unter Michaeladdition ein Thioether gebildet wird.

Weiterhin betrifft die Erfindung Verbindungen der Formel II, worin R und R¹ die dort angegebenen Bedeutungen haben.

Bevorzugt sind dabei Verbindungen, worin R der Seitenkette der Aminosäure Alanin, des Methioninsulfons oder der Cysteinsäure und ganz besonders der Aminosäure Alanin entspricht.

Ferner betrifft die Erfindung die Verwendung von Verbindungen nach Formel I zur Diagnose oder Therapie. Bevorzugt ist dabei die Verwendung in einem Enzymimmunoassay.

Schließlich betrifft die Erfindung die Verwendung einer Verbindung nach Formel II zur Herstellung einer Verbindung der Formel I.

Zur Herstellung der Verknüpfungsreagenzien nach Formel II werden z. B. nach dem Fachmann bekannten Verfahren Maleimidogruppen erzeugt, indem primäre Aminogruppen mit Maleinsäureanhydrid umgesetzt und die gebildeten Maleonylderivate unter Wasserabspaltung zu den Maleimidoderivaten (Maleoyl-Derivate) cyclisiert werden (Keller und Rudinger (1975); Helvetica Chimica Acta 58 , 531 - 541 und Rich et al. (1975); J.Med.Chem. 18, 1004 - 1010).
Die N-Hydroxysuccinimid- oder Sulfo-N-Hydroxisuccinimidestergruppen werden z. B. aus den Carboxylgruppen und N-Hydroxysuccinimid oder seinem Sulfonderivat unter Wasserabspaltung unter Zuhilfenahme von Reagenzien wie Carbodiimiden hergestellt (Anderson et al. (1964); J.Am.Chem.Soc. 86, 1839 ff).
Falls die Seitenketten R Gruppierungen tragen, die unter den Bedingungen der Bildung der Maleimidogruppe oder der Aktivestergruppen Nebenreaktionen eingehen, müssen dort entsprechende Substitutionen oder Modifizierungen zur Unterbindung dieser Nebenreaktionen vorgenommen werden. Bei Reagenzien ausgehend von der Aminosäure Methionin werden z. B. die oxidationsempfindlichen Thioetherfunktionen in die Sulfongruppen überführt, die zudem eine bessere Wasserlöslichkeit der resultierenden Verbindung mit sich bringen. Im Falle des Cystein bringt man z. B. eine gut wasserlösliche SH-Schutzgruppe wie die Acetamidomethyl (Acm)-Schutzgruppe oder noch vorteilhafter eine Oxidation der SH-Gruppe zur Sulfonsäuregruppe unter Bildung der Cysteinsäure, die als Sulfonsäuresalz zur Synthese des entsprechenden heterobifunktionellen Reagenzes eingesetzt werden kann, in Anwendung. Im Falle des Lysins oder Ornithins verwendet man für die Proteinmodifizierungsreagenzien bevorzugt polare, säurestabile Aminoschutzgruppen wie die Methylsulfonyloxicarbonyl (Msc)-Gruppe. Gegebenenfalls wird die Aminosäureseitenkettenschutzgruppe so ausgewählt, daß nach Verknüpfung der ersten zwei Kopplungspartner die Abspaltung der Seitenschutzgruppen ohne Zerstörung der für die Anwendung geforderten Eigenschaften der Kopplungspartner erfolgen kann. Beispiele sind die Verwendung der Msc-Schutzgruppe als Aminoschutzgruppe oder des Thiopyridylrestes als Thiolschutz. Beide Schutzgruppen sind in vielen Fällen bei Protein-Protein-Konjugaten ohne Schaden für die Proteine entfernbar. Im Falle des Thiolschutzes kann die freigesetzte Thiolfunktion benutzt werden, um eine dritte Kopplungskomponente einzuführen.

Die Verbindungen nach Formel II können nach dem Fachmann bekannten Methoden zur Herstellung von Konjugaten eingesetzt werden. Eine Übersicht über Methoden zur Herstellung von Proteinkonjugaten aus dem Bereich der Enzymimmunoassays ist z. B. in dem Reviewartikel von Ishikawa et al. gegeben (J.Immunoassay 4 (1983) 209 - 327). Methoden zur Herstellung von Immuntoxinen sind beispielsweise in dem Review von Ghose et al. aufgelistet (Methods of Enzymology, Vol. 93 (1983) pp. 280 - 333).

Reagenzien, die in der Brücke zwischen den beiden funktionellen Gruppen ein chirales C-Atom tragen, sind für Immunoassayanwendungen nicht beschrieben. Als einfachstes Glied dieser Reihe bietet sich ein Derivat an, das in der Brücke die Struktur -CH(CH₃)-CO- trägt. Ein solches Reagenz mit Maleimido- und Succinimidester- oder Sulfosuccinimidester-Gruppen zeigt erwartungsgemäß aufgrund des kleinen hydrophoben Anteils im Molekül gute Wasserlöslichkeit. Es läßt sich wie oben beschrieben aus der Aminosäure Alanin herstellen.

Reagenzien der Struktur II lassen sich vorteilhaft zur Verknüpfung von Immunoglobulinen und Markerenzymen für Immunoassays verwenden.

Dabei kann man beispielsweise in das Immunglobulin Thiolgruppen einführen (Ishikawa et al., 1983), in das Markerenzym Maleimidogruppen durch Umsetzung der Aminogruppen des Enzyms einführen und nach Abtrennen der Reagenzüberschüsse das modifizierte Immunglobulin mit dem modifizierten Enzym zur Kopplung umsetzen.

Bei einer anderen Art der Reaktionsführung lassen sich Maleimidogruppen durch Umsetzung der Aminogruppen des Immunglobulins mit Reagenzien der Struktur II einführen und mit im Markerenzym bereits vorhandenen Thiolgruppen (z. B. β-Galactosidase aus E. coli) oder Thiolgruppen, die durch Umsetzung mit Reagenzien wie 2-Iminothiolan eingeführt wurden, zur Kopplung umsetzen.

Besonders gezielt lassen sich die Konjugationen vom Bindungsort wie auch Stöchiometrie her durchführen, wenn Hinge-Thiole in Fab′ von Immunglobulinen mit Maleimidogruppen im Enzym, die durch Umsetzung des Enzyms mit Reagenzien der Struktur II erhältlich sind, zur Reaktion gebracht werden. Die Kopplung des Enzyms erfolgt dann an dem dem Paratop gegenüberliegenden Ende des Fab′. In einem analogen Fall lassen sich durch Reduktionsmittel Thiolgruppen im Hinge-Bereich von Immunglobulinen generieren und mit den Maleimidogruppen des Enzyms umsetzen. Überraschend war die gute Verknüpfbarkeit von Aminogruppen des einen Proteins mit SH-Gruppen der Aminosäure Cystein in dem anderen Protein bei der geringen Länge und geringen Flexibilität im Brückenteil des Vernetzungsreagenzes.

Die folgenden Beispiele sollen die Herstellung von Verbindungen der Struktur II und ihre Verwendung zur Herlung von Verbindungen der Struktur I erläutern.

### Beispiel 1

### Herstellung von Maleoyl-L-Alanin

L-Alanin (26,7 g; 0,30 mol) wird zu einer gerührten Lösung von Maleinsäuranhydrid (29,4 g; 0,30 mol) in Eisessig (300 ml) gegeben. Nach 3-stündigem Rühren wird das Reaktionsprodukt abfiltriert, getrocknet und aus Aceton/Hexan umkristallisiert. Nach Einengen der Mutterlauge können weitere Kristalle erhalten werden. Die Gesamtausbeute beträgt 33 g (60 %). Der Schmelzpunkt des Produktes liegt bei 142 - 144 °C.

### Beispiel 2

### Herstellung von Maleoyl-L-Alanin

Maleoyl-L-Alanin (4 g; 21 mmol) wird in trockenem Toluol (150 ml) suspendiert und nach Zusatz von Triethylamin (4 ml) 6 h unter Rückfluß mit Wasserabscheider unter Rühren gekocht. Dann wird die heiße Lösung vom orange gefärbten Rückstand abdekantiert und nach Abkühlung am Rotationsverdampfer zur Trockne eingeenet. Der zähe Rückstand wird in 50 ml Wasser, welches mit Salzsäure auf pH 3 eingestellt wurde, versetzt und unter jeweiliger Nachkorrektur des pH-Wertes 5 x mit jeweils 50 ml Ethylacetat bei pH 3 extrahiert. Die vereinigten Ethylacetat-Phasen werden am Rotationsverdampfer zur Trockne eingeengt, und das erhaltene Festprodukt wird über Nacht im Hochvakuum getrocknet.
Ausbeute: 1,25 g
¹H-NMR (200 MHz, CDCl₃; TMS): δ 1.65 (d, 3H, J = 7.4 Hz, CH₃), 4.82 (q, 1H, J = 7.4 Hz, CH), 6.74 (s, 2H, maleimido), 8.30 (bs, COOH).

### Beispiel 3

### Herstellung von Maleoyl-L-Alanin-N-Hydroxysuccinimidester (MAS)

Maleoyl-L-Alanin (200 mg; 1.2 mmol) und N-Hydroxysuccimid (150 mg; 1.3 mmol) werden in Tetrahydrofuran gelöst. Zu der auf 0°C abgekühlten Lösung wird unter Rühren eine Lösung von Dicyclohexylcarbodiimid (255 mg; 1.2 mmol) in Tetrahydrofuran (1 ml) zugetropft und über Nacht bei 4°C weitergerührt. Die erhaltene Suspension wird abfiltriert und das Filtrat am Rotationsverdampfer zur Trockne eingeengt. Der Rückstand wird in 2 ml Tetrahydrofuran resuspendiert und die Suspension über eine Glasfritte P4 filtriert. Das Filtrat wird am Rotationsverdampfer zur Trockne eingeengt, und das erhaltene Festprodukt wird im Hochvakuum getrocknet.
Ausbeute: 330 mg
¹H-NMR (200 MHz, CDCl₃; TMS): δ 1.77 (d, 3H, J = 7.4 Hz, CH₃), 2.84 (s, 4H, succinimidyl), 5.17 (q, 1H, J=7.4 Hz, CH), 6.80 (s, 2H, maleimido).

### Beispiel 4

### Herstellung eines Maleimido-Antikörpers

### a) Reagenzien

MAS hergestellt nach Beispiel 3 wird verwendet; Borsäure (E. Merck, Best. Nr. 165); Dioxan (E. Merck, Best. Nr. 3110); Lithiumhydroxyd (E. Merck, Best.Nr. 11652).

### b) Herstellung von Lösungen

- Lithiumboratpuffer, pH 8,5
   Borsäure (1,24 g) wird in einem Gemisch aus Wasser (80 ml) und Dioxan (20 ml) gerührt. Unter Auflösen der Borsäure wird durch Zugabe von festem Lithiumhydroxyd ein pH von 8,5 eingestellt.
- MAS-Lösung
   MAS (0,0124 g) wird in Dioxan (1 ml) gelöst.
- Phosphatpuffer pH 6,0
   Na-Dihydrogenphosphat-Monohydrat (41,4 g) und Titriplex (5,58 g) werden in Wasser (3 l) gelöst. Mit Natronlauge wird ein pH von 6,0 eingestellt.

### c) Herstellung des Maleimido-Antikörpers

Der zu konjugierende Antikörper (Konzentration 4 g/l in 1/15 M phosphatgepufferter physiologischer Kochsalzlösung, 25 ml) wird mit Lithiumboratpuffer (25 ml) vermischt. Zu der resultierenden Lösung mit einem pH von 7,5 wird unter Rühren 0,45 ml MAS-Lösung (entsprechend einem 30-fach molaren Überschuß von MAS gegenüber IgG) zugegeben. Nach 1-stündiger Inkubation bei Raumtemperatur wird der Reagenzüberschuß durch Gelfiltration an einer mit Phospatpuffer pH 6,0 äquilibrierten Sephadex G-25 Säule entfernt.

### Beispiel 5

### Herstellung einer Thiol-Peroxidase

### a) Reagenzien

Peroxidase (Reinheitsgrad I, Boehringer Mannheim, Best. Nr. 815462), 2-Iminothiolan-hydrochlorid (Sigma, Best. Nr. 6256), Methanol (E. Merck, Best. Nr. 6009), di-Na-Tetraborat-10-hydrat (E. Merck, Best. Nr. 6308)

### b) Lösungen

- Na-Borat-Lösung
   Di-Na-Tetraborat (0,952 g) wird in Wasser (100 ml) gelöst.
- Iminothiolan-Lösung
   2-Iminothiolan (0,688 g) wird in Methanol (5 ml) gelöst.

### c) Herstellung der Thiol-Peroxidase

Peroxidase (160 mg) wird in Na-Boratpuffer gelöst und mit Iminothiolan-Lösung vermischt. Nach 2-stündiger Inkubation des Reaktionsgemisches bei Raumtemperatur wird der Reagenzüberschuß durch Gelchromatographie an einer mit Phosphatpuffer pH 6,0 (Beispiel 4, Punkt b)) äquilibrierten Sephadex G-25 Säule entfernt.

### Beispiel 6

### Herstellung eines Peroxidase-Antikörper-Konjugates

### a) Reagenzien

Maleimido-Antikörper und Thiol-Peroxidase nach Beispiel 4) bzw. Beispiel 5), TRIS (E. Merck, Best. Nr. 8382)

### b) Lösungen

- TRIS-Puffer pH 7,4
   TRIS (18,17 g) wird in Wasser (3 l) gelöst und mit HCl ein pH von 7,4 eingestellt.

### c) Herstellung des Peroxidase-Antikörper-Konjugates

Maleimido-Antikörper-Lösung und Thiol-Peroxidase-Lösung werden entsprechend einem Molverhältnis von Antikörper zu Peroxidase von 1 : 5 vermischt. Nach 2-stündiger Inkubation bei Raumtemperatur wird die Reaktion durch Zugabe von 1/10 Volumen NEM-Lösung abgestoppt. Reagenzüberschüsse werden nach 30-minütiger Inkubation durch Gelchromatographie an einer Sephadex G-25 Säule, die mit TRIS-Puffer äquilibriert ist, entfernt.

### d) Eigenschaften des Konjugates

Bei der HPLC-Analyse durch Gelchromatographie auf einer DuPont GF-250-Säule wird kein freier Antikörper mehr nachgewiesen. Im Konjugatepeak wird durch optische Analyse ein molares Verhältnis von Peroxidase zu Antikörper von 2,5 ± 0,5 beim Vergleich verschiedener Präparationen mit polyklonalen Antikörpern gefunden. Bei monoklonalen Antikörpern kann das molare Verhältnis im Bereich von 1,5l bis 3,5 l schwanken. Das Konjugat kann in der für den jeweiligen Test spezifischen Gebrauchsverdünnung direkt für den Enzymimmunoassay eingesetzt werden. Eine Fraktionierung des Konjugates durch Gelchromatographie verbessert in Einzelfällen die spezifische Reaktion des Konjugates.

### Beispiel 7

### Herstellung einer Maleimido-Peroxidase

### a) Reagenzien und Lösungen

N-Ethylmorpholin, 98 %ig (Riedel de Haen, Best. Nr. 62050), N,N-Dimethylformamid (Riedel de Haen, Best. Nr. 15440), alle anderen Reagenzien und Lösungen wie in den Beispielen 4 - 6.

### b) Herstellung der Maleimido-Peroxidase

Peroxidase (1 g) wird in Wasser (6,5 ml) gelöst. Zu dieser Lösung wird Dioxan (3,5 ml) hinzugemischt. Mit Ethylmorpholin wird ein pH von 8,5 eingestellt. Diese Lösung wird mit einer Lösung von MAS (24 mg) in Dimethylformamid (0,5 ml) vermischt. Nach 1-stündiger Inkubation bei Raumtemperatur wird durch Gelfiltration an Sephadex G-25 in Phospatpuffer pH 6,0 umgepuffert.

### Beispiel 8

### Herstellung von Fab′-SH

Fab′ mit freien SH-Gruppen in der Hinge Region wird nach bekannten Methoden hergestellt (Ishikawa et al. (1983) J. Immunoassay 4, 209 - 327). Dazu wird IgG mit Pepsin zum (F(ab′)2 gespalten. Die Hinge-Disulfidbrücken im F(ab′)2 werden durch Thiol-Disulfidaustausch mit Mercaptoäthylamin zu Thiolen reduziert. Der Reaktionsansatz wird durch Gelfiltration vom Thiolüberschuß befreit und in Phospatpuffer pH 6,0 umgepuffert.

### Beispiel 9

### Herstellung eines Fab′-Peroxidase-Konjugates

Fab′-SH nach Beispiel 8 (100 mg in 20 ml Phosphatpuffer pH 6,0) wird mit Maleimidoperoxidase nach Beispiel 7 (500 mg in 10 ml Phosphatpuffer pH 6,0) vermischt und 1 Stunde bei 37°C inkubiert.
Das gebildete Konjugat wird von restlichem Fab′-SH und überschüssiger Peroxidase durch Gelchromatographie an Ultrogel ACA 44 (Fa. Pharmacia) abgetrennt. Elutionsmittel ist TRIS-Puffer pH 7,4.
Im Falle von Kaninchen Antikörpern eluiert der Hauptanteil des Konjugates in der Gelchromatographie entsprechend einem 1+1-Addukt aus Fab′ und Peroxidase. Das Produkt kann direkt in der für den Test spezifischen Gebrauchsverdünnung im Enzymimmunoassay eingesetzt werden.

## Patentansprüche (Patentansprüche für folgende(n) Vertragsstaat(en): AT, BE, CH, DE, FR, GB, IT, LI, LU, NL, SE)

1. Verbindung der Formel II 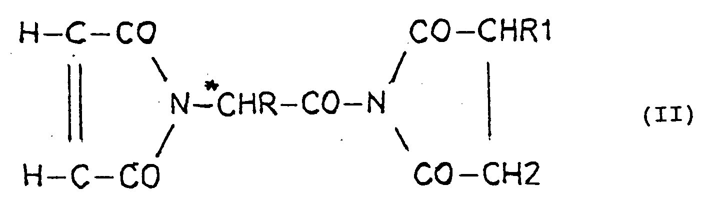 worin *C ein asymmetrisches Kohlenstoffatom ist,
R für die Seitenkette einer natürlichen Aminosäure, des Methioninsulfons oder der Cysteinsäure steht,
und
R¹ Wasserstoff oder einen Rest der Formel III bedeutet, 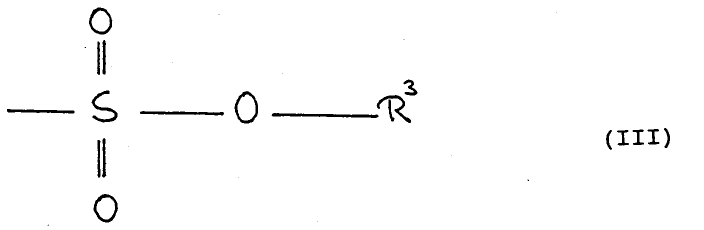 worin R³ Wasserstoff oder ein C₁ bis C₄ Alkyl ist.

2. Verbindungen nach Anspruch 1, dadurch gekennzeichnet, daß R der Seitenkette der Aminosäure Alanin, Methioninsulfon oder Cysteinsäure und bevorzugterweise der Aminosäure Alanin entspricht.

3. Konjugat der Formel I aus thiolhaltigen Biopolymeren und Effektorsubstanzen mit aminogruppentragenden Biopolymeren und Effektorsubstanzen 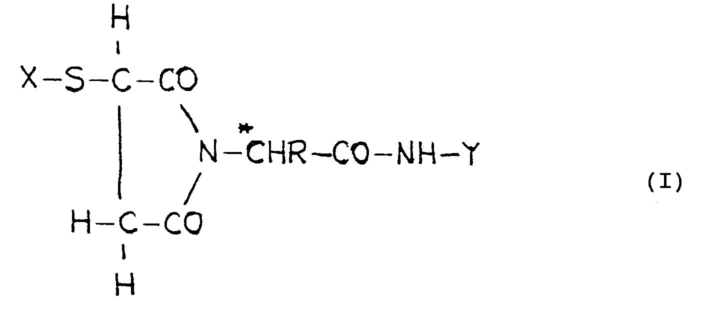 worin *C ein asymmetrisches Kohlenstoffatom ist,
R für die Seitenkette einer natürlichen Aminosäure, des Methioninsulfons oder der Cysteinsäure und
X für den Rest der Thiolkopplungskomponente und
Y für den Rest der Aminokopplungskomponente steht, dadurch gekennzeichnet, daß zur Kopplung eine Verbindung gemäß Anspruch 1 oder 2 eingesetzt wird.

4. Konjugate nach Anspruch 3, dadurch gekennzeichnet, daß die Thiol-Kopplungskomponente X-SH und die Aminokopplungskomponente Y-NH₂ jeweils Proteine sind.

5. Konjugate nach Anspruch 3, dadurch gekennzeichnet, daß
X ein Enzym, einen Antikörper oder ein Antikörperfragment und
Y ein Enzym, einen Antikörper oder ein Antikörperfragment bedeuten.

6. Verwendung eines Konjugates nach Anspruch 3 zur in-vitro-Diagnose.

7. Verwendung eines Konjugates nach Anspruch 3 in einem Enzymimmunoassay.

8. Verwendung einer Verbindung nach Anspruch 1 zur Herstellung eines Konjugates nach Anspruch 3.

9. Verfahren zur Herstellung einer Verbindung nach Anspruch 3, dadurch gekennzeichnet, daß in einem ersten Reaktionsschritt
durch Umsetzung einer Aminokomponente Y-NH₂ mit der funktionellen Aktivestergruppe einer Verbindung nach Anspruch 1, wobei bei der Herstellung der funktionellen Maleimido- oder Aktivestergruppe zu Nebenreaktionen führende Seitenketten durch den Fachmann bekannte Schutzgruppen geschützt sind
und
R¹ Wasserstoff oder einen Rest der Formel III bedeutet, 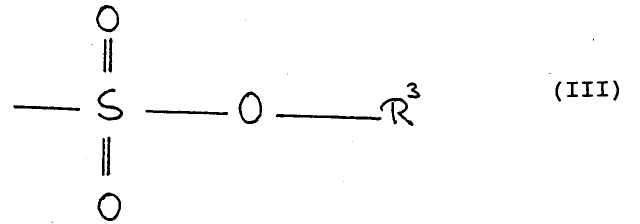 worin R³ Wasserstoff oder ein C₁ bis C₄ Alkyl ist, ein Amid
und in einem zweiten Reaktionsschritt
durch Umsetzung einer Thiolkomponente X-SH mit der Maleimidogruppe des im 1. Schritt entstandenen Amids der Verbindung der Formel II unter Michaeladdition ein Thiolether gebildet wird.

10. Konjugat gemäß Anspruch 3, dadurch gekennzeichnet, daß es aus einer Effektorsubstanz und einem Biopolymeren, insbesondere einem Antikörper oder einem Antikörperfragment besteht.

## Patentansprüche (Patentansprüche für folgende(n) Vertragsstaat(en): ES)

1. Verfahren zur Herstellung eines Konjugats der Formel I aus thiolhaltigen Biopolymeren und Effektorsubstanzen mit aminogruppentragenden Biopolymeren und Effektorsubstanzen 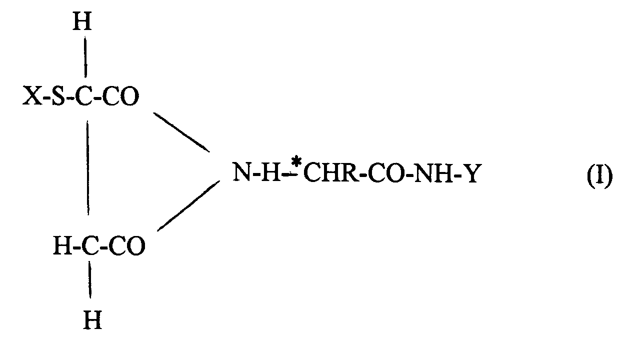 worin *C ein asymmetrisches Kohlenstoffatom ist, R für die Seitenkette einer natürlichen Aminosäure, des Methioninsulfons oder der Cysteinsäure steht,
X für den Rest der Thiolkopplungskomponente und
Y für den Rest der Aminokopplungskomponente steht dadurch gekennzeichnet, daß in einem ersten Reaktionsschritt durch Umsetzung einer Aminokomponente Y-NH₂ mit der funktionellen Aktivestergruppe einer Verbindung nach Anspruch 1, wobei bei der Herstellung der funktionellen Maleimido- oder Aktivestergruppe zu Nebenreaktionen führende Seitenketten durch den Fachmann bekannte Schutzgruppen geschützt sind und
R¹ Wasserstoff oder einen Rest der Formel III bedeutet, 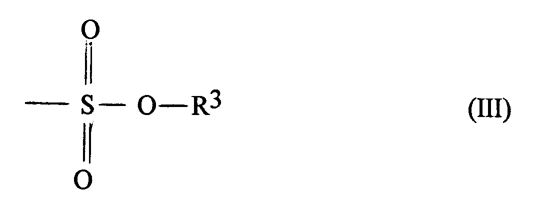 worin R³ Wasserstoff oder ein 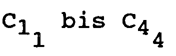 Alkyl ist, ein Amid und in einem zweiten Reaktionsschritt durch Umsetzung einer Thiolkomponente X-SH mit der Maleimidogruppe der Verbindung der Formel II unter Michaeladdition ein Thioether gebildet wird.

2. Verfahren nach Anspruch 1 dadurch gekennzeichnet, daß in Formel (I) R der Seitenkette der Aminosäure Alanin, Methioninsulfon oder Cysteinsäure und bevorzugterweise der Aminosäure Alanin entspricht.

3. Verfahren nach Anspruch 1 dadurch gekennzeichnet, daß die Thiol-Kopplungskomponente X-SH und die Aminokopplungskomponente Y-NH₂ jeweils Proteine sind.

4. Verfahren nach Anspruch 1 dadurch gekennzeichnet, daß X ein Enzym, einen Antikörper oder ein Antikörperfragement und Y ein Enzym, einen Antikörper oder ein Antikörperfragment bedeuten.

5. Verwendung eines nach Anspruch 1 hergestellten Konjugates zur in-vitro-Diagnose.

6. Verwendung eines nach Anspruch 1 hergestellten Konjugates in einem Enzymimmunoassay.

## Claims (Claims for the following Contracting State(s): AT, BE, CH, DE, FR, GB, IT, LI, LU, NL, SE)

1. A compound of the formula II 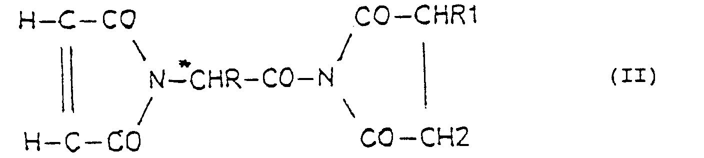 in which *C is an asymmetric carbon atom,
R is the side chain of a natural amino acid, of methionine sulfone or cysteic acid, and
R¹ is hydrogen or a radical of the formula III 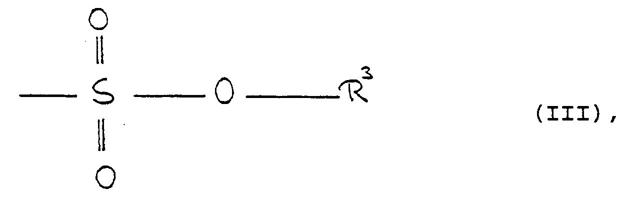 in which R³ is hydrogen or a C₁ to C₄ alkyl.

2. A compound as claimed in claim 1, wherein R corresponds to the side chain of the amino acid alanine, methionine sulfone or cysteic acid, and preferably of the amino acid alanine.

3. A conjugate of the formula I consisting of thiol-containing biopolymers and effector substances with amino-group-carrying biopolymers and effector substances 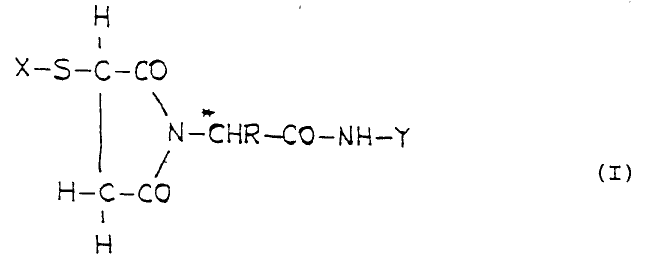 in which *C is an asymmetric carbon atom,
R is the side chain of a natural amino acid, of methionine sulfone or of cysteic acid, and
X is the radical of the thiol coupling component and
Y is the radical of the amino coupling component, wherein a compound as claimed in claim 1 or 2 is used for coupling.

4. A conjugate as claimed in claim 3, wherein the thiol coupling component X-SH and the amino coupling component Y-NH₂ are each proteins.

5. A conjugate as claimed in claim 3, wherein X is an enzyme, an antibody or a fragment of an antibody and Y is an enzyme, an antibody or a fragment of an antibody.

6. The use of a conjugate as claimed in claim 3 for in vitro diagnosis.

7. The use of a conjugate as claimed in claim 3 in an enzyme immunoassay.

8. The use of a compound as claimed in claim 1 for the preparation of a conjugate as claimed in claim 3.

9. A process for the preparation of a compound as claimed in claim 3, which comprises forming an amide in a first reaction step by reacting an amino component Y-NH₂ with the functional active ester group of a compound as claimed in claim 1, side chains which lead to side reactions in the preparation of the functional maleimido or active ester group being protected by protective groups known to those skilled in the art and
R¹ being hydrogen or a radical of the formula III 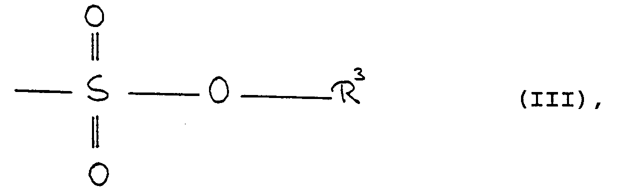 in which R³ is hydrogen or a C₁ to C₄ alkyl, an amide and forming a thioether in a second reaction step by Michael addition, by reacting a thiol component X-SH with the maleimido group of the amide, produced in the 1st step, of the compound of the formula II

10. The conjugat of Claim 3, characterized in that the conjugat consists of a biopolymer and an effector substance wherein preferably the biopolymer is an antibody or a fragment of an antibody.

## Claims (Claims for the following Contracting State(s): ES)

1. A process for the preparation of a conjugate of the formula I consisting of thiol-containing biopolymers and effector substances with amino-group-carrying biopolymers and effector substances 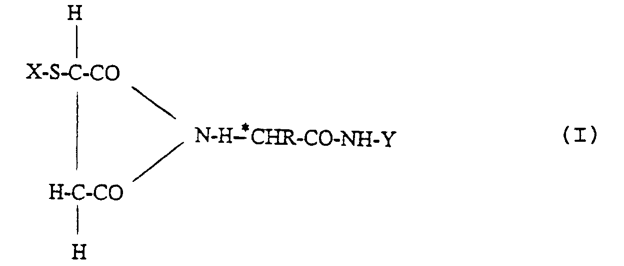 in which *C is an asymmetric carbon atom, R is the side chain of a natural amino acid, of methionine sulfone or of cysteic acid,
X is the radical of the thiol coupling component and Y is the radical of the amino coupling component, which comprises forming an amide in a first reaction step by reacting an amino component Y-NH₂ with the functional active ester group of a compound as claimed in claim 1, side chains which lead to side reactions in the preparation of the functional maleimido or active ester group being protected by protective groups known to those skilled in the art and
R¹ being hydrogen or a radical of the formula III 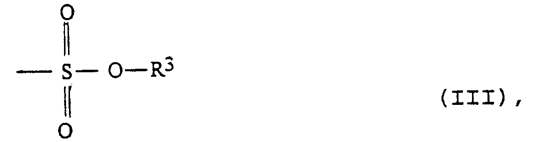 in which R³ is hydrogen or a C₁ to C₄ alkyl, an amide and forming a thioether in a second reaction step by Michael addition, by reacting a thiol component X-SH with the maleimido group of the compound of the formula II.

2. The process as claimed in claim 1, wherein, in formula (I), R corresponds to the side chain of the amino acid alanine, methionine sulfone or cysteic acid, and preferably of the amino acid alanine.

3. The process as claimed in claim 1, wherein the thiol coupling component X-SH and the amino coupling component Y-NH₂ are each proteins.

4. The process as claimed in claim 1, wherein X is an enzyme, an antibody or a fragment of an antibody and Y is an enzyme, an antibody or a fragment of an antibody.

5. The use of a conjugate prepared as claimed in claim 1 for in vitro diagnosis.

6. The use of a conjugate prepared as claimed in claim 1 in an enzyme immunoassay.

## Revendications (Revendications pour l'(les) Etat(s) contractant(s) suivant(s): AT, BE, CH, DE, FR, GB, IT, LI, LU, NL, SE)

1. Composé de formule II 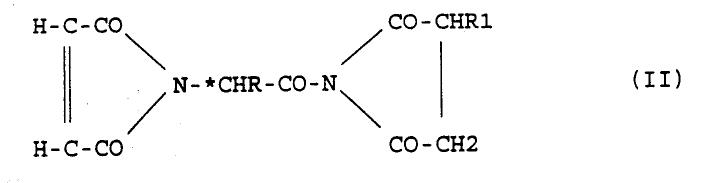 dans laquelle *C est un atome de carbone asymétrique,
R représente la chaîne latérale d'un acide aminé naturel, de la méthionine sulfone ou de la cystéine, et
R1 représente l'hydrogène ou un reste de formule III 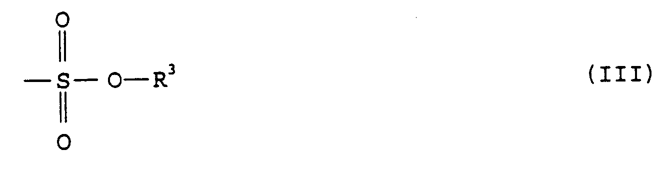 dans laquelle R³ est l'hydrogène ou un alkyle en C₁-C₄.

2. Composés selon la revendication 1, caractérisées en ce que R correspond à la chaîne latérale de l'acide aminé alanine, de la méthionine sulfone ou de la cystéine, et de façon préférée, de l'acide aminé alanine.

3. Conjugué de formule I dérivé de biopolymères et d'effecteurs à base de thiol avec des biopolymères comportant des groupes amino et des effecteurs 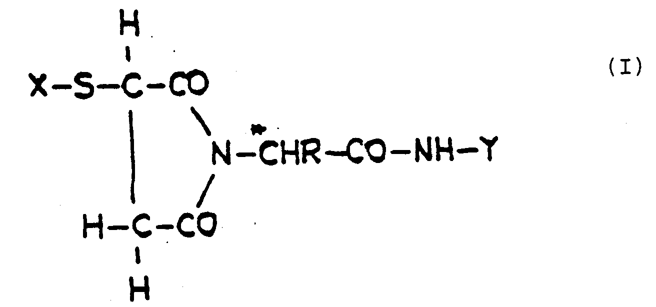 dans lequel *C est un atome de carbone asymétrique,
R représente la chaîne latérale d'un acide aminé naturel, de la méthionine sulfone ou de la cystéine,
X représente le reste du composant de couplage thiol et Y représente le reste du composant de couplage amino, caractérisé en ce qu'un composé selon la revendication 1 ou la revendication 2 est utilisé pour le couplage.

4. Conjugué selon la revendication 3, caractérisé en ce que le composant de couplage thiol X-SH et le composant de couplage amino Y-NH₂ sont des protéines, respectivement.

5. Conjugué selon la revendication 3, caractérisé en ce que X désigne une enzyme, un anticorps ou un fragment d'anticorps, et
Y désigne une enzyme, un anticorps ou un fragment d'anticorps.

6. Utilisation d'un conjugué selon la revendication 3 pour le diagnostic in vitro.

7. Utilisation d'un conjugué selon la revendication 3 dans un dosage immuno-enzymatique.

8. Utilisation d'un composé selon la revendication 1 pour la préparation d'un conjugué selon la revendication 3.

9. Procédé de préparation d'un composé selon la revendication 3, caractérisé en ce que, dans une première étape réactionnelle, un amide est formé en faisant réagir un composant amino Y-NH₂ avec le groupe ester activé d'un composé selon la revendication 1, les chaînes latérales pouvant conduire à des réactions secondaires lors de la préparation du groupe fonctionnel maléimido ou ester activé étant protégées par des groupes protecteurs connus des spécialistes et
R1 représentant l'hydrogène ou un reste de formule III 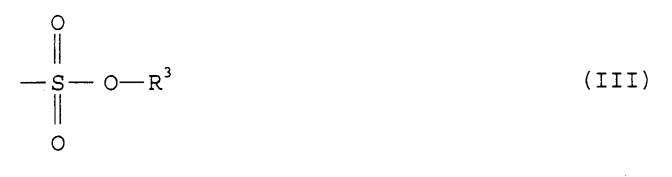 dans laquelle R³ est l'hydrogène ou un alkyle en C₁-C₄, et un thio éther est formé dans une seconde étape réactionnelle en faisant réagir un thiol X-SH avec le groupe maléimido de l'amide du composé de formule II obtenu lors de la première étape par addition de Michael.

10. Conjugué selon la revendication 3, caractérisé en ce qu'il se compose d'un effecteur et d'un biopolymère, notamment d'un anticorps ou d'un fragment d'anticorps.

## Revendications (Revendications pour l'(les) Etat(s) contractant(s) suivant(s): ES)

1. Procédé de préparation d'un conjugué de formule I dérivé de biopolymères et d'effecteurs à base de thiol avec des biopolymères comportant des groupes amino et des effecteurs 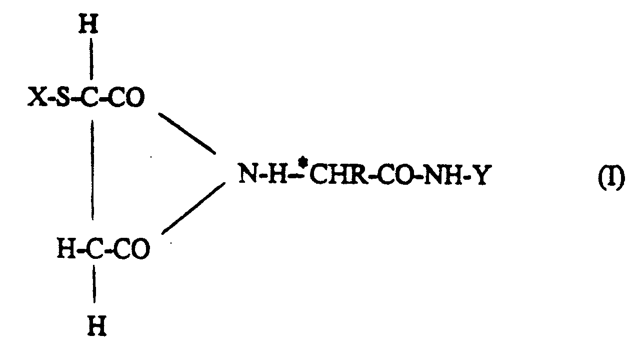 dans lequel *C est un atome de carbone asymétrique,
R représente la chaîne latérale d'un acide aminé naturel, de la méthionine sulfone ou de la cystéine,
X représente le reste du composant de couplage thiol et
Y représente le reste du composant de couplage amino, caractérisé en ce que, dans une première étape réactionnelle un amide est formé en faisant réagir un composant amino Y-NH₂ avec le groupe ester activé d'un composé selon la revendication 1, les chaînes latérales pouvant conduire à des réactions secondaires lors de la préparation du groupe fonctionnel maléimido ou ester activé étant protégées par des groupes protecteurs connus des spécialistes
et
R1 représentant l'hydrogène ou un reste de la formule III 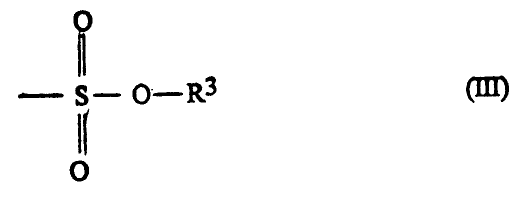 dans lequel R³ est l'hydrogène ou un alkyle en C₁-C₄, et un thio éther est formé dans une seconde étape réactionnelle en faisant réagir un composant thiol X-SH avec le groupe maléimido de l'amide du composé de la formule II obtenu lors de la première étape par addition de Michael.

2. Procédé selon la revendication 1, caractérisé en ce que dans la formule (I) R correspond à la chaîne latérale de l'acide aminé alanine, de la méthionine sulfone ou de la cystéine, et de façon préférée, de l'acide aminé alanine.

3. Procédé selon la revendication 1, caractérisé en ce que le composant de couplage thiol X-SH et le composant de couplage amino Y-NH₂ sont des protéines, respectivement.

4. Procédé selon la revendication 1, caractérisé en ce que X désigne une enzyme, un anticorps ou un fragment d'anticorps, et Y désigne une enzyme, un anticorps ou un fragment d'anticorps.

5. Utilisation d'un conjugué préparé selon la revendication 1 pour le diagnostic in vitro.

6. Utilisation d'un conjugué préparé selon la revendication 1 dans un dosage immuno-enzymatique.
